# NEUE EUROPÄISCHE PATENTSCHRIFT

(11) **EP 2 782 844 B2**
(45) Veröffentlichungstag und Bekanntmachung des Hinweises auf die Entscheidung über den Einspruch: **11.12.2019**
(45) Hinweis auf die Patenterteilung: 27.07.2016
(21) Anmeldenummer: 12790468.8
(22) Anmeldetag: 08.11.2012
(51) Int. Cl.: B65D 47/20

(54) **SPENDER ZUM AUSTRAG VON PHARMAZEUTISCHEN FLÜSSIGKEITEN**
DISPENSER FOR DISPENSING PHARMACEUTICAL LIQUIDS
DISTRIBUTEUR DESTINÉ À DÉLIVRER DES LIQUIDES PHARMACEUTIQUES

(30) Priorität: 21.11.2011 DE 102011086755
(43) Veröffentlichungstag der Anmeldung: 01.10.2014
(73) Patentinhaber: Aptar Radolfzell GmbH, 78315 Radolfzell (DE)
(72) Erfinder: WOCHELE, Matthias, 78239 Rielasingen-Worblingen (DE)
(74) Vertreter: Patentanwaltskanzlei Cartagena
(86) Internationale Anmeldenummer: PCT/EP2012/072138
(87) Internationale Veröffentlichungsnummer: WO 2013/075951

(56) Entgegenhaltungen:
- EP-A1- 0 864 371
- WO-A1-2009/130411
- WO-A1-2010/013131
- WO-A1-2012/013894
- WO-A1-2012/013894
- CA-A1- 2 492 255
- DE-U1-202004 013 331
- FR-A1- 2 934 572
- US-A- 5 013 459
- US-A- 5 154 325
- US-A- 6 129 248
- US-A1- 2008 264 827
- US-B1- 6 227 413

## Beschreibung

### Anwendungsgebiet und Stand der Technik

Die Erfindung betrifft einen Spender zum Austrag von pharmazeutischen Flüssigkeiten nach den Oberbegriffen von Anspruch 1 und 2.

Aus der gattungsbildenden WO 2010/013131 A1 ist ein gattungsbildender Spender bekannt. Der dort vorgeschlagene Spender verfügt über ein Kopfteil aus einem antibakteriell wirkenden Material.

Ähnliche Spender sind aus dem Stand der Technik allgemein bekannt. Sie verfügen über ein Flüssigkeitsreservoir, aus dem Flüssigkeit in Richtung der Austrittsöffnung gefördert werden kann, wobei dies über viele verschiedene Mechanismen erfolgen kann. So kann das Flüssigkeitsreservoir als Quetschflasche ausgebildet sein, deren Inhalt durch Verformung der Wandungen unter Druck gesetzt werden kann. Auch eine separate Pumpeinrichtung ist verwendbar. Diese Spender verfügen über ein Auslassventil. Hierbei handelt es sich um ein Ventil welches im Flüssigkeitspfad unmittelbar vor der Auslassöffnung angeordnet ist. Zumeist handelt es sich um ein druckabhängig öffnendes Auslassventil, welches durch Druckbeaufschlagung der Flüssigkeit im Flüssigkeitsspeicher oder einer hieraus entnommenen Teilcharge geöffnet wird und selbsttätig wieder schließt, sobald der entsprechende Überdruck gegenüber der Umgebung entfällt. Grundsätzlich sind hier jedoch auch andere Ventiltypen verwendbar. So kann beispielsweise vorgesehen sein, dass die Flüssigkeit im Flüssigkeitsreservoir permanent unter Druck steht und die Handhabung des Spenders über eine Handhabe erfolgt, deren manuelle Betätigung das Auslassventil öffnet.

Im Bereich dieser Spender führt das Auslassventil dazu, dass nach Schließen desselben keinerlei Flüssigkeit, welche bis in den zweiten Teilabschnitt des Auslasskanals gelangt ist oder welche in der Umgebung der Austrittsöffnung außerhalb des Auslasskanals verblieben ist, zurück in den Spender eingesogen werden kann. Eine mögliche Kontamination des Inhaltes des Flüssigkeitsspeichers durch zurückgesogene Flüssigkeitsreste wird dadurch verhindert.

Die Restflüssigkeit verbleibt daher in einem von außen zugänglichen Bereich. Hier ist allerdings eine Kontamination zu befürchten ist.

Um eine Kontamination insbesondere bei Konservierungsmittel freien Flüssigkeiten zu vermeiden, ist es aus dem Stand der Technik grundsätzlich bereits bekannt, antibakterielle Oberflächen am Spender zu verwenden, die in Kontakt mit der Flüssigkeit gelangen. Beschrieben ist derartiges beispielsweise in der US 5,232,687, deren Ausführungsbeispiel einen Spender beschreibt, dessen Auslassventil antibakteriell ausgebildet ist.

Es hat sich jedoch in der Vergangenheit gezeigt, dass die Ausgestaltung von Flächen in antibakterieller Hinsicht auch problematische Seiteneffekte haben kann. Je nach Art der Ausgestaltung der antibakteriellen Flächen ist zu verzeichnen, dass sich Bestandteile der entsprechend ausgebildeten Oberfläche von dieser lösen und in die Flüssigkeit gelangen. Sie werden dann zusammen mit der Flüssigkeit ausgetragen. Dies ist in medizinischer Hinsicht nachteilig.

### Aufgabe und Lösung

Aufgabe der Erfindung ist es daher, einen gattungsgemäßen Spender zur Verfügung zu stellen, bei dem die Problematik der Kontamination der Flüssigkeit gemildert wird.

Erfindungsgemäß wird dies durch einen erfindungsgemäßen Spender nach den Ansprüchen 1 und 2 erzielt.

Bei einem erfindungsgemäßen Spender ist somit vorgesehen, dass gezielt nur solche Oberflächen antibakteriell ausgebildet sind, die sich nicht im Dauerkontakt mit der Gesamtheit der im Spender enthaltenen Flüssigkeit befinden. Stattdessen soll die antibakterielle Wirkung, die von den entsprechend ausgebildeten Oberflächen ausgeht, ausschließlich jene Flüssigkeit betreffen, die bereits jenseits des Auslassventils angeordnet ist und die daher zum einen nicht zurück in das Flüssigkeitsreservoir gelangen kann und zum anderen aufgrund ihrer Anordnung jenseits des Auslassventils einer Kontamination in besonderem Maße ausgesetzt sein kann.

Bei einem erfindungsgemäßen Spender ist die Flüssigkeit, die sich im Flüssigkeitsspeicher und den Flüssigkeitsbereichen zwischen dem Flüssigkeitsspeicher und dem Auslassventil befindet, dadurch vor Kontamination geschützt, dass das Auslassventil und ein möglicher Lufteinlass derart ausgebildet sind, dass keine Kontamination in den Spender hineingelangt. Auf eine spezielle Bakterien tötende Gestaltung innerhalb des Spenders kann zugunsten der Vermeidung der oben genannten Seiteneffekte daher verzichtet werden. Lediglich die Flüssigkeitsreste jenseits des Auslassventils werden dadurch vor Kontamination geschützt, dass dort Bakterien tötende oder deren Wachstum verhindernder Oberflächengestaltungen vorgesehen sind. Es wurde festgestellt, dass durch die Kombination dieser Maßnahmen eine besonders gute Qualität der Flüssigkeit gewährleistet werden kann.

Beispielhaft sind folgende Möglichkeiten gegeben, um die antibakterielle Ausgestaltung der betreffenden Oberflächen zu erzielen: So kann die Oberfläche mit Silber in seiner metallischen Form oder in Form einer Silberverbindung, mit Silbersalzen, Silberchloriden, Silbersulfadiazin Silberoxiden oder einer Silberlegierung versehen sein. Auch Nanometall-partikel, insbesondere Nanosilberpartikel könne Verwendung finden. Weiterhin zweckmäßig ist die Verwendung eines Biduanid-Derivats, Chlorhexidindiacetats oder Chlorhexidindigluconats. Des Weiteren können quaternäre Ammoniumverbindungen wie Benzalkoniumchlorid und Stearalkoniumchlorid zur Erzielung der keimfeindlichen Wirkung verwendet werden. Auch existieren zweckmäßig verwendbare organische Farbstoffe mit antiseptischer Wirkung, die vorliegend verwendet werden können. Hierzu zählen beispielsweise Toluidinblau, Methylenblau, Gentianviolet sowie Acridin und hiermit verwandte Wirkstoffe wie Acridinorange und Acridingelb sowie Ethacridinlactat. Auch keimtötende Polymere wie Polyhexanid kommen in Frage. Eine weitere Möglichkeit besteht darin, dass Material des entsprechenden Bauteils oder die aufgebrachte Beschichtung mit Additiven zu versehen, die metallorganische Stoffe mit ionisierender Wirkung beinhalten. Derartige Additive werden beispielsweise unter dem Namen "Sterione" von einem gleichnamigen Unternehmen vertrieben.

Die entsprechende Ausgestaltung der jenseits des Auslassventils vorgesehenen Oberflächen führt dazu, dass der hier verbleibende Flüssigkeitsrest, insbesondere der Resttropfen, der nach Abgabe eines Tropfens mittels eines Tropfenspenders verbleibt, wirksam von einer Kontamination abgehalten wird.

Von besonderer Bedeutung ist die erfindungsgemäße Ausgestaltung des als Tropfenspender ausgebildeten Spenders, der eine die Austrittsöffnung umgebende Tropfenbildungsfläche aufweist, die außenseitig von einer Abrisskante begrenzt wird. Bei einem solchen Tropfenspender verbleibt nahezu unvermeidbar an der Tropfenbildungsfläche der genannte Resttropfen aufgrund physikalischer Gegebenheiten. Dies ist insbesondere im Zusammenhang mit Tropfenspendern für die Einbringung in das Auge eine Gefahr, da sich hier Verunreinigungen der Flüssigkeit besonders kritisch auswirken.

Insbesondere betrifft dies Tropfenspender, deren Flüssigkeitsreservoir mit einer Flüssigkeit befüllt ist, die der Behandlung von Augenkrankheiten dient. Hierbei kann es sich beispielsweise um eine Flüssigkeit mit einem der folgenden Wirkstoffe handeln: Ranibizumab, Latanoprost, Travoprost, Ciclosporin, Bimatoprost, Olopatadin, Dorzolamid, Moxifloxacin, Brimonidin, Hyaluronsäure, Brinzolamid, Dexamethason, Levofloxacin, Gatifloxacin, Zellulose und Verteporfin.

Zur Ausgestaltung einer jenseits des Auslassventils angeordneten Oberfläche ist die erfindungsgemäße Gestaltung gemäß Anspruch 1 vorgesehen, bei der der zweite Teilabschnitt des Auslasskanals zumindest abschnittsweise von einem antibakteriellen ausgebildeten Einsatz gebildet wird, welcher als gegenüber dem ersten Teilabschnitt und den Funktionsteilen des Ventils separates Bauteil ausgebildet ist. Der Einsatz ist in Form eines rohrförmigen oder ringförmigen Bauteils vorgesehen, dessen Innenwandung einen Teil des zweiten Teilabschnitts des Auslasskanals bildet. Die Verwendung eines solchen vergleichsweise kleinen Bauteils, welches in Hinblick auf die antibakterielle Ausgestaltung des Spenders beschaffen ist, gestattet es, mit vergleichsweise geringem Aufwand ansonsten baugleiche Spender mit oder ohne einen antibakteriell ausgebildeten Einsatz zu verwenden oder einen Einsatz zu verwenden, welcher auf die verwendete Flüssigkeit in besonders guter Art und Weise angepasst ist. Somit können annähernd baugleiche Spender für verschiedene Anwendungszwecke Verwendung finden und sich voneinander primär lediglich hinsichtlich des Einsatzes unterscheiden.

Der genannte Einsatz wird entgegen der Austrittsrichtung der Flüssigkeit durch die Austrittsöffnung in eine an die Außenform des Einsatzes angepasste Ausnehmung des Gehäuses eingeschoben. Hier gehalten werden kann er mittels eines beispielsweise durch eine Rastverbindung herstellbaren Formschlusses oder auch rein reibschlüssig.

Der Einsatz bildet dabei erfindungsgemäß bei der Gestaltung nach Anspruch 1 durch eine nach außen weisende Stirnseite gleichzeitig einen Teil der Tropfenbildungsfläche oder die gesamte Tropfenbildungsfläche, sodass nicht nur im Auslasskanal verbleibende Flüssigkeitsreste, sondern auch der Resttropfen im Bereich der Tropfenbildungsfläche in unmittelbarem Kontakt mit der antibakteriell ausgebildeten Oberfläche des Einsatzes angeordnet ist.

Statt einer solchen Lösung, bei der der Einsatz als Träger der antibakteriellen Oberflächen in eine Ausnehmung eingeschoben wird, kann bei einer alternativen Ausgestaltung gemäß Anspruch 2 erfindungsgemäß vorgesehen sein, wenn das Gehäuse einen antibakteriell ausgebildeten Aufsatz aufweist, der im Bereich der Austragöffnung auf ein vom zweiten Teilabschnitt des Auslassventils durchdrungenes Gehäuseteil aufgeschoben ist. Bei dieser zweiten Gestaltung ist somit ein in sich funktionstauglicher Spender vorgesehen, der auch ohne den genannten Aufsatz zum Austragen von Flüssigkeit geeignet ist. Dieser kann durch den genannten aufgeschobenen Aufsatz jedoch um eine Funktionalität in Hinblick auf die antibakteriellen Eigenschaften ergänzt werden. Zur Festlegung des Aufsatzes an das korrespondierende Gehäuseteil kann eine Rastverbindung vorgesehen sein. Von Vorteil ist es jedoch, wenn die Außenseite dieses als Träger agierenden Gehäuseteils zumindest abschnittsweise zylindrisch oder leicht konisch (Winkel < 3°) ausgebildet ist, sodass der Aufsatz rein reibschlüssig in der vorgesehenen Position gehalten ist.

Der Aufsatz bildet vorzugsweise die gesamte Tropfenbildungsfläche, wodurch neben dem Vorteil der antibakteriellen Ausgestaltung der Austausch des Einsatzes es auch gestattet, die Formgebung der Tropfenbildungsfläche fallweise zu beeinflussen und dabei den Spender im Übrigen unverändert zu belassen.

Weitere Vorteile und Aspekte der Erfindung ergeben sich außer aus den Ansprüchen auch aus den nachfolgend beschriebenen Ausführungsbeispielen der Erfindung. Dabei zeigen:
Figur 1 einen erfindungsgemäßen Spender in einer Gesamtdarstellung,
Figur 1a die Auslassbaugruppe des Spenders der Fig. 1 mit aufgesetzter Kappe und
Figuren 2 und 3 verschiedene Auslassbaugruppen für den Spender der Figur 1.

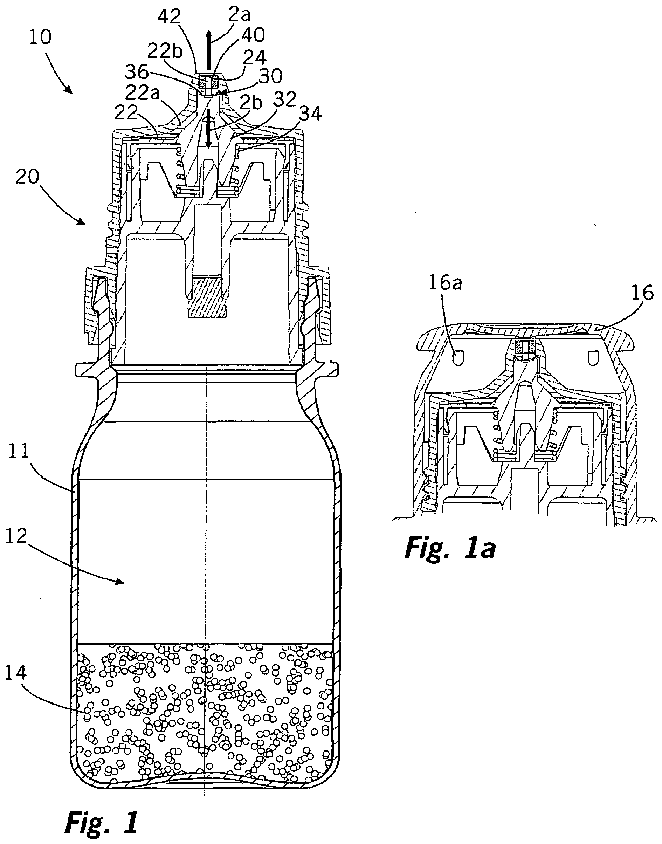

Figur 1 zeigt zunächst einen erfindungsgemäßen Spender in einer Gesamtdarstellung.

Dieser Spender 10 verfügt über einen von einem Behälterkörper 11 begrenzten Flüssigkeitsspeicher 12. Auf den Behälterkörper 11 ist eine Auslassbaugruppe 20 aufgesetzt und mittels einer Rastverbindung befestigt. Diese Auslassbaugruppe 20 dient dem Zweck, durch einen Auslasskanal 22 hindurch Flüssigkeit vom Flüssigkeitsspeicher 12 bis zu einer Austragöffnung 24 zu leiten. In der Darstellung der Fig. 1 ist aufgrund der Schnittebene lediglich ein letztes Teilstück dieses Auslasskanals 22 dargestellt.

Die Verwendung des Spenders 10 erfolgt derart, dass dieser in einen Überkopflage mit nach unten weisender Austragöffnung 24 gebracht wird. Anschließend werden Wandungen des Behälterkörpers 11 zusammengedrückt, um die Flüssigkeit 14 im Flüssigkeitsspeicher 12 mit einem Druck zu beaufschlagen. Dieser Druck bewirkt ein Öffnen eines Auslassventils 30, welches zwischen dem Flüssigkeitsspeicher 12 und der Auslassöffnung 24 vorgesehen ist. Das Auslassventil 30 umfasst einen Ventilkörper 32, welcher mittels einer Ventilfeder 34 permanent in Richtung einer Ventilfläche 36 gedrückt wird. Sobald der Flüssigkeitsdruck in einem Teilabschnitt 22a des Auslasskanals 22 vor dem Auslassventil 30 ausreichend hoch ist, wird der Ventilkörper 32 durch diesen Druck in Richtung des Pfeils 2b verlagert und gibt den Weg für die Flüssigkeit in den zweiten Teilabschnitt 22b des Auslasskanals 22 frei. Die Flüssigkeit wird dann in Richtung des Pfeils 2a durch die Austragöffnung 24 hindurchgeleitet.

Der dargestellte Spender 10 ist als Tropfenspender für ophthalmische Zwecke vorgesehen. Er weist daher die Austragöffnung 24 umgebend eine Tropfenbildungsfläche 40 auf, die durch eine Abrisskante 42 nach außen begrenzt wird.

In der von Tropfenspendern üblichen und unvermeidbaren Art bleibt nach dem Austrag von Flüssigkeit in Tropfenform ein Rest der Flüssigkeit, der so genannte Resttropfen, an der genannten Tropfenbildungsfläche 40 sowie im zweiten Teilabschnitt 22b des Auslasskanals 22 zurück. Ein Rückfluss in den ersten Teilabschnitt 22a des Auslasskanals 22 oder in den Flüssigkeitsspeicher 12 ist aufgrund des druckabhängig öffnenden Auslassventils 30 nicht möglich.

Da gewünscht ist, den Spender mit einer konservierungsmittelarmen oder konservierungsmittelfreien Flüssigkeit zu verwenden, ist der jenseits des Auslassventils 30 im zweiten Teilabschnitt 22b des Auslasskanals 22 und an der Tropfenbildungsfläche 40 verbleibende Flüssigkeitsrest besonders gefährdet, durch Keime verunreinigt zu werden. Dies gilt umso mehr, wenn zum Zwecke eines raschen Abtrocknens eine Kappe 16 des Spenders 10 mit Belüftungsöffnungen 16a versehen ist, die eine dauerhafte Verbindung zwischen der Tropfenbildungsfläche 40 und einer äußeren Umgebung schaffen. Solche Belüftungsöffnungen 16a sind in Fig. 1a dargestellt.

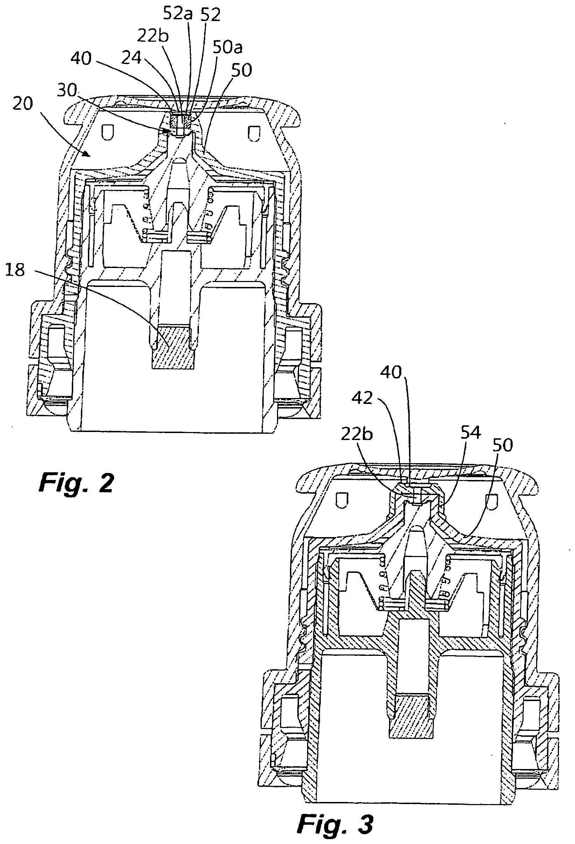

Um dennoch in Oberflächenbereichen jenseits des Auslassventils 30 keine Bakterienbildung in inakzeptablem Maße befürchten zu müssen, sind die in den Figuren 2 und 3 vorgeschlagenen Gestaltungen der Auslassbaugruppe 20 von Nutzen.

Bei der Ausgestaltung der Figur 2, welche der der Figur 1 entspricht, ist in einem Außenbauteil 50, welches vom Flüssigkeitskanal 22 durchdrungen ist, eine zylindrische Aussparung 50a vorgesehen. In dieser zylindrischen Aussparung 50a ist ein Einsatz 52 eingesetzt, welcher die Form eines Rohrabschnittes aufweist. Die Außenfläche des Einsatzes 52 ist derart an die Aussparung 50a des Gehäusebauteils 50 angepasst, dass durch Hineindrücken des Einsatzes 52 in die Aussparung 50a eine reibschlüssige Verbindung geschaffen wird, durch die der Einsatz 52 sicher in seiner in Figur 2 dargestellten Position verbleibt. Stattdessen könnte jedoch eine formschlüssig wirkende Verbindung vorgesehen sein.

Die obere Stirnseite 52a des Einsatzes 52 bildet eine ringförmige Fläche, welcher Teil der Tropfenbildungsfläche 40 ist. Sie bildet den überwiegenden Teil der Tropfenbildungsfläche 40.

Der Einsatz 52 ist in seiner Gesamtheit aus einem antibakteriellen, also Bakterienwachstum vermindernden, Material oder aus einem Bakterien tötenden Material ausgebildet. Hierbei handelt es sich vorzugsweise um einen Kunststoff, der mit antibakteriell wirkenden Additiven versehen ist.

Nach Beendigung eines Austragvorgangs verbleiben der Resttropfen sowie der im zweiten Teilabschnitt 22b zurückgebliebenen Flüssigkeitsreste in großer Nähe zur antibakteriell wirkenden Fläche. Die Gefahr der übermäßigen Verkeimung dieses Flüssigkeitsrestes ist daher gering.

Gleichzeitig gewährleistet die Anordnung des Einsatzes 52 jedoch, dass keinerlei Flüssigkeit, die noch vor dem Auslassventil 30 angeordnet ist, in Kontakt mit antibakteriell wirkenden Flächen gelangt. Die noch nicht am Auslassventil 30 vorbeigeströmte Flüssigkeit wird alleine durch die Filterung der einströmenden Luft mittels eines Filters 18 sowie durch die dichte Ausgestaltung des Auslassventils 30 von Keimen freigehalten. Die negative Wirkung von antibakteriellen Additiven, die sich im Dauerkontakt mit der Flüssigkeit im Flüssigkeitsspeicher oder im ersten Teilabschnitt 22a befinden, bleibt hiermit somit aus. Es besteht keine Gefahr, dass Additive oder anderweitige Bestandteile aus antibakteriell ausgebildeten Oberflächen in die Flüssigkeit gelangen und dort negative Wirkung entfalten.

Bei der Ausgestaltung der Figur 3 ist statt des Einsatzes 52 ein Aufsatz 54 vorgesehen. Wiederum ist lediglich der Aufsatz 54, der auf ein Gehäusebauteil 50, welches vom Austrittskanal 22 durchdrungen wird, aufgesetzt ist, aus einem antibakteriell wirkenden Kunststoff gefertigt. Ein Teil des zweiten Teilabschnitts 22b des Auslasskanals 22 sowie die gesamte Tropfenbildungsfläche 40 mitsamt ihrer außenseitig die Tropfenbildungsfläche 40 abschließenden Abrisskante 42 besteht aus dem antibakteriell wirkenden Kunststoff. Die Flüssigkeit innerhalb des Flüssigkeitsspeichers bleibt wiederum frei von möglicherweise aus dem Kunststoff austretenden Additiven.

## Patentansprüche

1. Spender (10) zum Austrag von pharmazeutischen Flüssigkeiten (14) mit
- einem Gehäuse (11, 50, 52, 54, 56),
- einem innerhalb des Gehäuses angeordneten Flüssigkeitsreservoir (12),
- einer Austrittsöffnung (24), durch die hindurch die Flüssigkeit (14) in eine umgebende Atmosphäre abgegeben werden kann und
- einem Auslasskanal (22), der das Flüssigkeitsreservoir (12) mit der Austrittsöffnung (24) verbindet,
wobei
- das Gehäuse (50) im Bereich von Oberflächen, die bestimmungsgemäß mit der Flüssigkeit in Kontakt gelangen, antibakteriell ausgebildet ist und
- der Spender als Tropfenspender ausgebildet ist,
**dadurch gekennzeichnet, dass**
- der Spender (10) ein druckabhängig öffnendes oder manuell betätigbares Auslassventil (30) aufweist, welches im Auslasskanal (22) angeordnet ist und welches bei geschlossenem Ventil (30) den Auslasskanal in einen ersten Teilabschnitt (22a) vor dem Auslassventil (30) und einen zweiten Teilabschnitt (22b) hinter dem Auslassventil (30) unterteilt, - ausschließlich Oberflächen im Bereich des zweiten Teilabschnitts (22b) und/oder einer Außenoberfläche (40, 50, 52, 54, 56) des Gehäuses diese antibakterielle Ausbildung aufweisen,
- der Spender die Austrittsöffnung (24) umgebend eine Tropfenbildungsfläche (40) aufweist, die außenseitig von einer Abrisskante (42) begrenzt wird, und
- der zweite Teilabschnitt (22b) des Auslasskanals (22) zumindest abschnittsweise von einem antibakteriell ausgebildeten Einsatz (52) gebildet wird, welcher als gegenüber dem ersten Teilabschnitt (22a) und den Funktionsteilen des Ventils (30) separates rohrförmiges oder ringförmiges Bauteil ausgebildet ist, dessen Innenwandung einen Teil des zweiten Teilabschnitts (22b) des Auslasskanals (22) bildet, und
- der Einsatz (52) entgegen der Austrittsrichtung (2b) der Flüssigkeit durch die Austrittsöffnung (24) in eine an die Außenform des Einsatzes angepasste Ausnehmung (50a) des Gehäuses (50) eingeschoben ist, und
- der Einsatz (52) die Tropfenbildungsfläche (40) zumindest abschnittsweise definiert.

2. Spender (10) zum Austrag von pharmazeutischen Flüssigkeiten (14) mit
- einem Gehäuse (11, 50, 52, 54, 56),
- einem innerhalb des Gehäuses angeordneten Flüssigkeitsreservoir (12),
- einer Austrittsöffnung (24), durch die hindurch die Flüssigkeit (14) in eine umgebende Atmosphäre abgegeben werden kann,
- einem Auslasskanal (22), der das Flüssigkeitsreservoir (12) mit der Austrittsöffnung (24) verbindet und wobei
- das Gehäuse (50) im Bereich von Oberflächen, die bestimmungsgemäß mit der Flüssigkeit in Kontakt gelangen, antibakteriell ausgebildet ist, und
- der Spender (10) als Tropfenspender ausgebildet ist,
**dadurch gekennzeichnet, dass**
- der Spender (10) ein druckabhängig öffnendes oder manuell betätigbares Auslassventil (30) aufweist, welches im Auslasskanal (22) angeordnet ist und welches bei geschlossenem Ventil (30) den Auslasskanal in einen ersten Teilabschnitt (22a) vor dem Auslassventil (30) und einen zweiten Teilabschnitt (22b) hinter dem Auslassventil (30) unterteilt,
- ausschließlich Oberflächen im Bereich des zweiten Teilabschnitts (22b) und/oder einer Außenoberfläche (40, 50, 52, 54, 56) des Gehäuses diese antibakterielle Ausbildung aufweisen, und
- das Gehäuse einen antibakteriell ausgebildeten Aufsatz (54) umfasst, der im Bereich der Austrittsöffnung (24) auf ein vom zweiten Teilabschnitt (22b) durchdrungenes Gehäuseteil (50) aufgeschoben ist und
- der Aufsatz (54) eine die Austrittsöffnung (24) umgebende Tropfenbildungsfläche (40) zumindest abschnittsweise bildet, die außenseitig von einer Abrisskante (42) begrenzt wird.

3. Spender nach einem der vorstehenden Ansprüche,
**dadurch gekennzeichnet, dass**
die antibakterielle Ausbildung durch ein in den Kunststoff eingebrachtes Additiv erzeugt wird.

4. Spender nach einem der Ansprüche 1 oder 2,
**dadurch gekennzeichnet, dass**
die antibakterielle Ausbildung dadurch erzeugt ist, dass der zweiten Teilabschnitts des Auslasskanals und/oder einer Außenoberfläche des Gehäuses mit einer antibakteriell wirkenden Beschichtung versehen ist oder einer physikalischen Behandlung unterzogen wurde.

## Claims

1. Dispenser (10) for dispensing pharmaceutical liquids (14), having
- a housing (11, 50, 52, 54, 56),
- a liquid reservoir (12), which is arranged within the housing,
- an outlet opening (24), through which the liquid (14) can be discharged into a surrounding atmosphere and
- an outlet channel (22), which connects the liquid reservoir (12) to the outlet opening (24),
wherein
- the housing (50) is of antibacterial design in the region of surfaces which are intended to come into contact with the liquid and
- the dispenser is designed in the form of a droplet dispenser,
**characterized in that**
- the dispenser (10) has an outlet valve (30) which can be opened in a pressure-dependent manner, or can be actuated manually, is arranged in the outlet channel (22) and, with the valve (30) closed, subdivides the outlet channel into a first portion (22a) upstream of the outlet valve (30) and a second portion (22b) downstream of the outlet valve (30),
- it is exclusively surfaces in the region of the second portion (22b) and/or in the region of an outer surface (40, 50, 52, 54, 56) of the housing which have this antibacterial design,
- the dispenser has a droplet-forming surface (40) enclosing the outlet opening (24) which is bounded on the outside by a separation edge (42), and
- the second portion (22b) of the outlet channel (22) is formed, at least in part, by an antibacterial insert (52), which is designed in the form of a tubular or ring-shaped component which is separate from the first portion (22a) and the functional parts of the valve (30), the inner wall of the component forming a part of the second portion (22b) of the outlet passage (22), and
- the insert (52) is pushed through the outlet opening (24), counter to the outlet direction (2b) of the liquid, into a recess (50a) of the housing (50), said recess being adapted to the external shape of the insert, and
- the insert (52) defines the droplet-forming surface (40) at least in part.

2. Dispenser (10) for dispensing pharmaceutical liquids (14), having
- a housing (11, 50, 52, 54, 56),
- a liquid reservoir (12), which is arranged within the housing,
- an outlet opening (24), through which the liquid (14) can be discharged into a surrounding atmosphere,
- an outlet channel (22), which connects the liquid reservoir (12) to the outlet opening (24), and wherein
- the housing (50) is of antibacterial design in the region of surfaces which are intended to come into contact with the liquid, and
- the dispenser is designed in the form of a droplet dispenser,
**characterized in that**
- the dispenser (10) has an outlet valve (30) which can be opened in a pressure-dependent manner, or can be actuated manually, is arranged in the outlet channel (22) and, with the valve (30) closed, subdivides the outlet channel into a first portion (22a) upstream of the outlet valve (30) and a second portion (22b) downstream of the outlet valve (30),
- it is exclusively surfaces in the region of the second portion (22b) and/or in the region of an outer surface (40, 50, 52, 54, 56) of the housing which have this antibacterial design, and
- the housing comprises an antibacterial attachment (54), which is pushed, in the region of the outlet opening (24), onto a housing part (50) through which the second portion (22b) penetrates, and
- the attachment (54) has a droplet-forming surface (40) enclosing the outlet opening (24) which is bounded on the outside by a separation edge (42).

3. Dispenser according to one of the preceding claims,
**characterized in that**
the antibacterial design is created by an additive introduced into the plastics material.

4. Dispenser according to one of Claims 1 or 2,
**characterized in that**
the antibacterial design is created **in that** the second portion of the outlet channel and/or of an outer surface of the housing is provided with an antibacterially active coating or has been subjected to a physical treatment.

## Revendications

1. Distributeur (10) pour distribuer des liquides pharmaceutiques (14), comprenant :
- un boîtier (11, 50, 52, 54, 56),
- un réservoir de liquide (12) disposé à l'intérieur du boîtier,
- une ouverture de sortie (24) à travers laquelle le liquide (14) peut être délivré dans une atmosphère environnante et
- un canal de sortie (22) qui relie le réservoir de liquide (12) à l'ouverture de sortie (24),
- le boîtier (50) étant réalisé sous forme antibactérienne dans la région de surfaces qui viennent en contact avec le liquide de manière conforme aux prescriptions et
- le distributeur étant réalisé sous forme de compte-gouttes,
**caractérisé en ce que**
- le distributeur (10) présente une valve de sortie (30) s'ouvrant en fonction de la pression ou pouvant être commandée manuellement, laquelle est disposée dans le canal de sortie (22) et, lorsque la valve (30) est fermée, divise le canal de sortie en une première portion partielle (22a) devant la valve de sortie (30) et une deuxième portion partielle (22b) derrière la valve de sortie (30),
- exclusivement des surfaces dans la région de la deuxième portion partielle (22b) et/ou d'une surface extérieure (40, 50, 52, 54, 56) du boîtier présentent cette réalisation antibactérienne,
- le distributeur présente une surface de formation de goutte (40) entourant l'ouverture de sortie (24), laquelle surface est limitée du côté extérieur par une arête de décrochage (42), et
- la deuxième portion partielle (22b) du canal de sortie (22) est formée au moins en partie par un insert (52) réalisé sous forme antibactérienne, lequel est réalisé sous forme de composant tubulaire ou annulaire séparé par rapport à la première portion partielle (22a) et aux pièces fonctionnelles de la valve (30), la paroi interne duquel forme une partie de la deuxième portion partielle (22b) du canal de sortie (22), et
- l'insert (52) est introduit à l'encontre de la direction de sortie (2b) du liquide à travers l'ouverture de sortie (24) dans un évidement (50a) du boîtier (50) adapté à la forme extérieure de l'insert, et
- l'insert (52) définit au moins en partie la surface de formation de goutte (40).

2. Distributeur (10) pour distribuer des liquides pharmaceutiques (14), comprenant :
- un boîtier (11, 50, 52, 54, 56),
- un réservoir de liquide (12) disposé à l'intérieur du boîtier,
- une ouverture de sortie (24) à travers laquelle le liquide (14) peut être délivré dans une atmosphère environnante,
- un canal de sortie (22) qui relie le réservoir de liquide (12) à l'ouverture de sortie (24) et
- le boîtier (50) étant réalisé sous forme antibactérienne dans la région de surfaces qui viennent en contact avec le liquide de manière conforme aux prescriptions, et
- le distributeur (10) étant réalisé sous forme de compte-gouttes,
**caractérisé en ce que**
- le distributeur (10) présente une valve de sortie (30) s'ouvrant en fonction de la pression ou pouvant être commandée manuellement, laquelle est disposée dans le canal de sortie (22) et, lorsque la valve (30) est fermée, divise le canal de sortie en une première portion partielle (22a) devant la valve de sortie (30) et une deuxième portion partielle (22b) derrière la valve de sortie (30),
- exclusivement des surfaces dans la région de la deuxième portion partielle (22b) et/ou d'une surface extérieure (40, 50, 52, 54, 56) du boîtier présentent cette réalisation antibactérienne, et
- le boîtier comprend un capuchon (54) réalisé sous forme antibactérienne qui est poussé sur une partie de boîtier (50) traversée par la deuxième portion partielle (22b) dans la région de l'ouverture de sortie (24) et
- le capuchon (54) forme au moins en partie une surface de formation de goutte (40) entourant l'ouverture de sortie (24), laquelle surface est limitée du côté extérieur par une arête de décrochage (42).

3. Distributeur selon l'une quelconque des revendications précédentes,
**caractérisé en ce que**
la réalisation antibactérienne est produite par un additif introduit dans la matière plastique.

4. Distributeur selon l'une quelconque des revendications 1 et 2,
**caractérisé en ce que**
la réalisation antibactérienne est produite par le fait que la deuxième portion partielle du canal de sortie et/ou d'une surface extérieure du boîtier est pourvue d'un revêtement à action antibactérienne ou a été soumise à un traitement physique.
